(19) **Europäisches Patentamt** / **European Patent Office** / **Office européen des brevets**

(11) **EP 4 518 825 B1**

(12) **EUROPEAN PATENT SPECIFICATION**

(45) Date of publication and mention
of the grant of the patent:
**14.01.2026   Bulletin 2026/03**

(21) Application number: **23725839.7**

(22) Date of filing: **04.05.2023**

(51) International Patent Classification (IPC):
*A61H 1/02 (2006.01)*        *A63B 21/005 (2006.01)*
*A63B 23/12 (2006.01)*       *A63B 21/16 (2006.01)*
*A63B 71/00 (2006.01)*       *A63B 23/035 (2006.01)*
*A63B 22/00 (2006.01)*       *A63B 21/00 (2006.01)*
*A63B 23/16 (2006.01)*       *A63B 21/008 (2006.01)*

(52) Cooperative Patent Classification (CPC):
**A63B 21/00178; A61H 1/0274; A61H 1/0285;
A63B 21/00181; A63B 21/0058; A63B 21/16;
A63B 21/4047; A63B 21/4049; A63B 22/00;
A63B 23/03525; A63B 23/03533; A63B 23/03541;
A63B 23/1209; A63B 71/0054;** A61H 2201/0119;
(Cont.)

(86) International application number:
**PCT/IB2023/054653**

(87) International publication number:
**WO 2023/214349 (09.11.2023 Gazette 2023/45)**

(54) **DEVICE FOR BILATERAL REHABILITATION**

VORRICHTUNG ZUR BILATERALEN REHABILITATION

DISPOSITIF DE RÉÉDUCATION BILATÉRALE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **06.05.2022   IT 202200009317**

(43) Date of publication of application:
**12.03.2025   Bulletin 2025/11**

(73) Proprietor: **Consiglio Nazionale Delle Ricerche
00185 Roma (IT)**

(72) Inventors:
• **MALOSIO, Matteo
  00185 Roma (IT)**
• **LAVIT NICORA, Matteo
  00185 Roma (IT)**
• **REDAELLI, Davide
  00185 Roma (IT)**
• **TAURO, Giovanni
  00185 Roma (IT)**

(74) Representative: **Leihkauf, Steffen Falk
Jacobacci & Partners S.p.A.
Via Senato, 8
20121 Milano (IT)**

(56) References cited:
**EP-A2- 3 599 002      EP-B1- 1 734 912
US-A- 4 337 050        US-A1- 2017 304 137**

(52) Cooperative Patent Classification (CPC): (Cont.)
A61H 2201/013; A61H 2201/0157; A61H 2201/018;
A61H 2201/10; A61H 2201/1215; A61H 2201/1238;
A61H 2201/1635; A61H 2201/1671;
A61H 2201/1676; A61H 2201/5012;
A61H 2201/5061; A61H 2201/5069;
A61H 2201/5092; A61H 2201/5097; A61H 2230/04;
A61H 2230/60; A61H 2230/65; A63B 21/0085;

A63B 21/4035; A63B 23/16; A63B 2022/0092;
A63B 2022/0094; A63B 2071/0072; A63B 2209/08;
A63B 2209/10; A63B 2213/004; A63B 2220/16;
A63B 2220/36; A63B 2220/54; A63B 2220/58;
A63B 2220/805; A63B 2220/806; A63B 2225/055;
A63B 2225/20; A63B 2225/50; A63B 2230/04;
A63B 2230/10; A63B 2230/60; A63B 2230/65

**Description**

The present invention relates to a bilateral rehabilitation device for the upper and/or lower limbs of a user (patient) by means of the interaction between the user and the device for carrying out various motor gestures of the user's upper or lower limbs, involving different joints, in order to contribute to the recovery of the functionality and motor coordination of the upper or lower limbs and to facilitate a so-called cerebral plasticity mechanism.

Bilateral rehabilitation of the upper and lower limbs is based on the assumption that during coordinated bilateral movements, the movement of the non-paretic limb can support the stimulation of the movement of the paretic limb at the cerebral level. Such a rehabilitation approach can be effective for example in cases of cerebral palsy, stroke, or other cases of hemiparesis.

Bilateral rehabilitation devices, especially those intended for autonomous use in the home, should ideally meet the following requirements: portability, structural and user simplicity (ergonomics), adaptability to different anthropometric sizes, versatility for carrying out different types of exercises (monolateral/bilateral), low costs.

The availability of increasingly low-cost mechatronic technologies on the one hand, and the growing demand for rehabilitation solutions and equipment for out-of-hospital and home use, in order to intensify patient care, on the other hand, are stimulating the research and development of innovative rehabilitation equipment which reconciles the need to limit costs with the need for versatility of use.

However, the rehabilitation devices currently on the market do not allow use in multiple different configurations. Therefore, rehabilitation plans with different motor gestures require the purchase and use of multiple specific devices. It would instead be desirable to have a single device capable of supporting multiple different motor gestures.

The bimanual rehabilitation devices listed below are known to the inventors and can be considered a background art for the present invention.

The BATRAC® device and Rocker® device are non-configurable manual rehabilitation devices which allow only one movement of the hands, but not also of other joints of the entire upper limb, for example the shoulder, elbow.

Other devices known to the inventors or under development allow wider movement of the upper limb, but constrain the movements of the upper limbs along fixed, unchangeable directions.

The Tailwind® device is a so-called "passive", non-motorized device which determines the movement of the upper limbs by means of two linear guides.

The Reha-Slide Duo® device is similar to the Tailwind® device and does not allow orienting the movement directions of the guides in an independent manner, but allows mutually constraining the movements of the two manipulandums.

The Bimanual Lifting Rehabilitator® device allows adjusting a lifting intensity depending on the movements and forces exchanged between the user's hands and interface devices (handles), amplifying the lifting/raising force. However, even the Bimanual Lifting Rehabilitator® device does not allow any reconfigurations to carry out multiple different motor gestures.

Rehabilitation devices with accessories for guiding and carrying out movements with two upper limbs, such as the Iron Arm, are also known, but the possible movements are limited in one plane and independent movements of the two upper limbs are not possible.

Devices are also known (Hand Robotic Rehabilitation Device®) with two motorized cranks with adjustable rotation axes, structurally divided into several separate housings and therefore classifiable as a system consisting of several distinct devices with the related installation dimensions.

More complex bimanual rehabilitation devices are also known, for example bilateral exoskeletons, for example EXO-UL7®, which allow a series of different motor gestures but require a vertical wall installation in the hospital.

Document EP1734912B1 discloses a device comprising all the technical features set out in the preamble of claim 1.

Therefore, the purpose of the present invention is to provide a device for the bilateral rehabilitation of the limbs (understood as upper and/or lower limbs) having features such as to be usable also in the domestic field, structurally simple, less cumbersome with respect to the devices of the known art, as well as configurable for carrying out different types of movement.

This and other objects are achieved by a bilateral rehabilitation device according to claim 1. Advantageous and preferred embodiments are the subject of the dependent claims.

In order to better understand the invention and appreciate the advantages thereof, some non-limiting embodiments thereof will be described below by way of non-limiting example with reference to the accompanying drawings, in which:

> figures 1, 2, 3 are perspective views of a bilateral rehabilitation device according to an embodiment,
> figures 4, 5, 6, 7 are front (figure 4), bottom (figure 5), side (figures 6, 7) views of the rehabilitation device in figure 1,
> figures 8A, 8B, 8C are front views of the rehabilitation device in three different rotary axis orientation configurations, according to an embodiment,
> figure 9 is an exploded view of the rehabilitation device, according to an embodiment,
> figures 10.1 and 10.2 are perspective views of the rehabilitation device with a first set of grip accessories in two different gripping orientation configurations, such accessories being suitable for both the upper limbs and the lower limbs,

figures 10.3 and 10.4 are perspective views of the rehabilitation device with a second set of grip accessories in two different gripping orientation configurations and in two different rotation axis orientation configurations, such accessories being suitable for both the upper limbs and the lower limbs,

figure 10.5 is a perspective view of the rehabilitation device with a third set of grip accessories (cranks, pedals) in a gripping orientation configuration of a plurality of gripping orientation configurations, and in a rotation axis configuration of a plurality of different rotation axis orientation configurations, such accessories being suitable for both the upper limbs and the lower limbs,

figure 10.6 is a perspective view of the rehabilitation device with a fourth set of grip accessories (linkage) in a gripping orientation configuration of a plurality of gripping orientation configurations, and in a rotation axis configuration of a plurality of different rotation axis orientation configurations,

figure 10.7 is a perspective view of the rehabilitation device with a fourth set of grip accessories similar to the third set of handle accessories (cranks, pedals) in a different gripping orientation configuration, and with an electronic screen, such accessories being suitable for both the upper limbs and the lower limbs,

figure 10.8 is a perspective view of the rehabilitation device with a further set of mutually different grip accessories, in a reconfigurable gripping orientation configuration, and with an electronic screen, such accessories being suitable for both the upper limbs and the lower limbs,

figure 10.9 is a diagrammatic view of a translational grip accessory, which is operable by a rotary-translational motion conversion mechanism, such an accessory being suitable for both the upper limbs and the lower limbs,

figure 11 is an exploded view of a mechanism for adjusting the orientation of a grip accessory of the rehabilitation device, according to an embodiment,

figure 12 shows a motorized drive mechanism of the rehabilitation device according to an embodiment,

figure 13 is a partially exploded view of a portion of the motorized drive mechanism in figure 12,

figures 14.1, 14.2, 14.3, 14.4, 14.6 show control block diagrams of the bilateral rehabilitation device according to embodiments, in which:

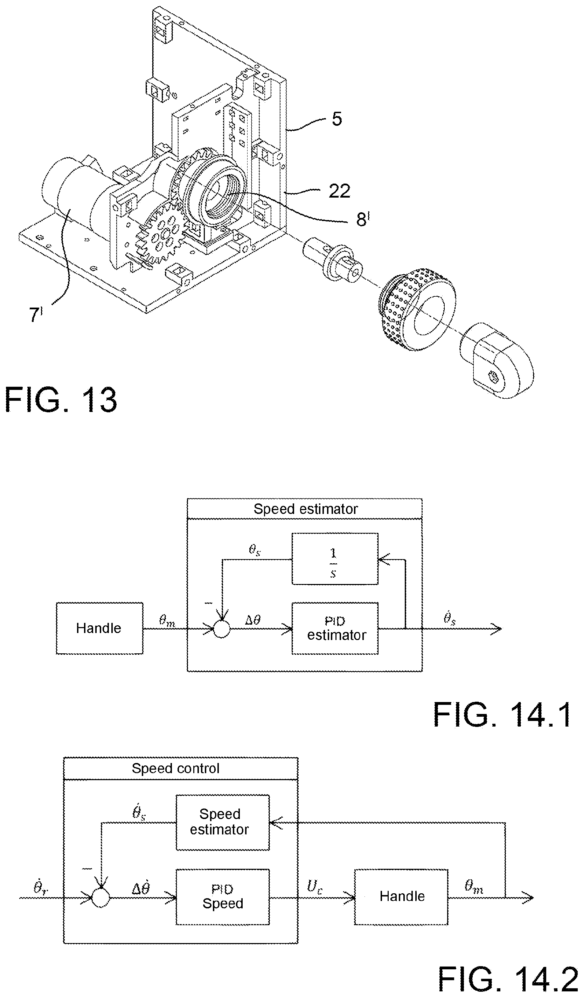

- fig. 14.1 shows an angular speed estimator module,
- fig. 14.2 shows an angular speed control module

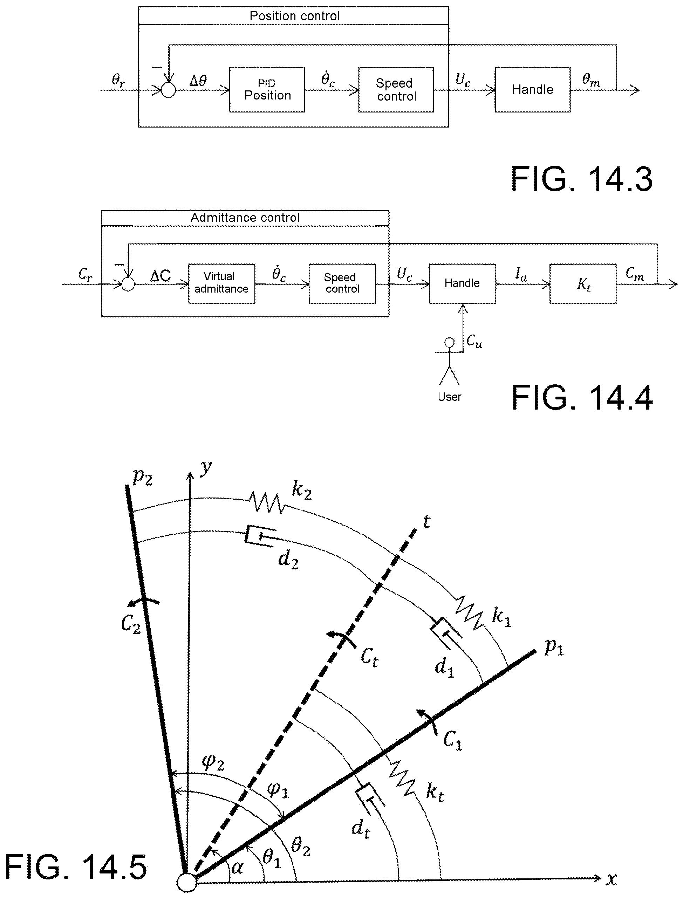

- fig. 14.3 shows an angular position control module
- fig. 14.4 shows an admittance control module,
- fig. 14.5 shows a bimanual (or similarly bi-foot or generically bi-arm or bilateral) virtual mechanics model,

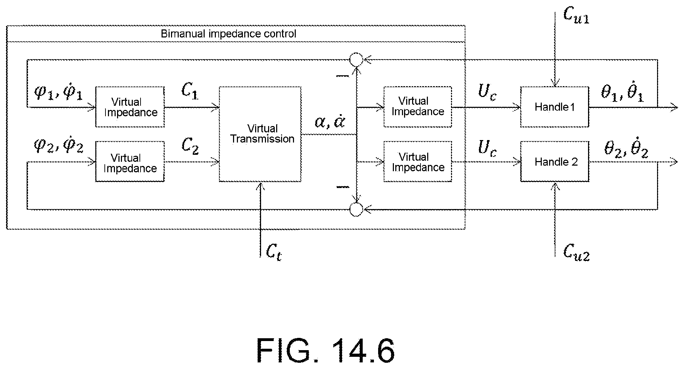

- fig. 14.6 shows a bi-lateral impedance control module implementing the virtual mechanics model of figure 14.5,

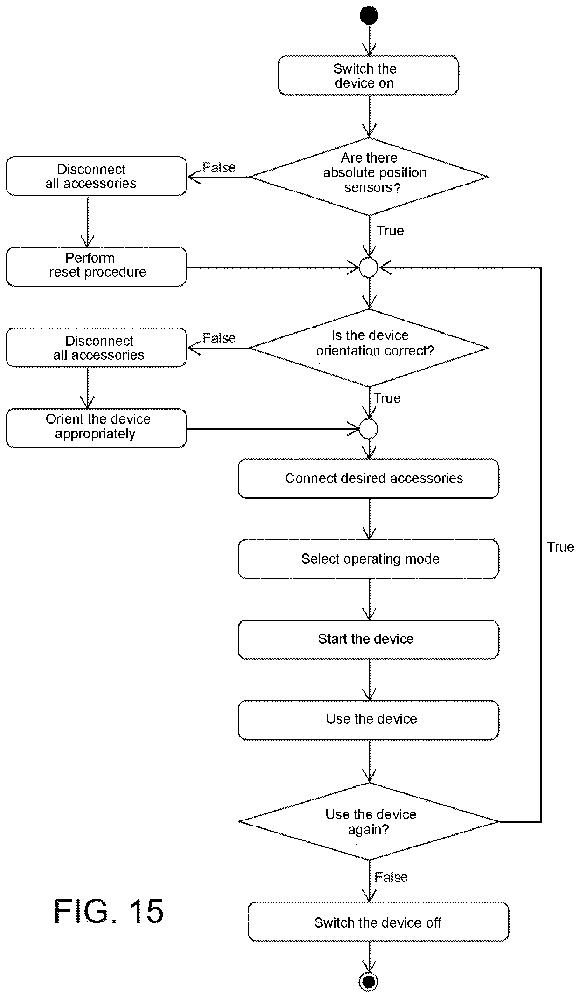

figure 15 is a block diagram of an operating method of the rehabilitation device according to an embodiment.

## General description of the invention

According to an aspect of the invention, a bilateral rehabilitation device 1 for a user's limbs (understood as upper or lower) comprises:

- a connecting base 2 with connecting members 3 for a stationary connection of the connecting base 2 to a support 4,
- a main body 5 connected to the connecting base 2,
- a first electric motor 7' and a second electric motor 7" supported by the main body 5,
- a first rotary interface 8' supported by the main body 5 in a first position and which is rotatably operable about a first rotation axis 15' by the first motor 7', and a second rotary interface 8" supported by the main body 5 in a second position spaced from the first position and rotatably operable about a second rotation axis 15" by the second motor 7",
- a set 12 of grip accessories 12.1, 12.2, ..., 12.n each having a grip portion 13 (intended to be gripped by the user) and a coupling portion 14 which is reversibly couplable integral in rotation to one of the first 8' and second 8" rotary interfaces, respectively,
- an electronic control device 11 in signal connection with the first motor 7' and with the second motor 7" and comprising:

  - first position detecting means 9' for detecting an angular position of the first rotary interface 8' with respect to the main body 5,
  - second position detecting means 9" for detecting an angular position of the second rotary interface 8" with respect to the main body 5,
  - first torque detecting means 10' for detecting a torque applied to the first rotary interface 8',
  - second torque detecting means 10" for detecting a torque applied to the second rotary interface 8",

in which the electronic control device 11 is configured to control the rotary movements and torques applied to the first 8' and second 8" rotary interfaces depending on the angular positions and/or the torques detected by the first and second position detecting means 9', 9" and the first and second torque detecting means 10', 10",

in which the main body 5 is connected to the connecting base 2 by means of an adjustable and lockable joint 6 which allows an adjustment and locking of the position of the main body 5 with respect to the connecting base 2 in at least three (or at least two or three) different body positions with different orientations of the first 15' and second 15" rotation axes.

In this context the feature "different body positions with different orientations of the first 15' and second 15" rotation axes" means that the orientation of the first rotation axis 15' changes when the body position changes and also the orientation of the second rotation axis 15" changes when the body position changes. Instead, the orientation of the first rotation axis 15' with respect to the orientation of the second rotation axis 15" does not necessarily change. Both the first 15' and second 15" rotation axes can maintain, for example, a mutually fixed relative position or can be parallel or coaxial, i.e., coincident.

By virtue of the configuration of the rehabilitation device 1, it can be used in a domestic environment, structurally simple and compact, as well as configurable for carrying out different types of limb movement.

The adjustment of position and orientation of two rotation axes of two different spaced apart rotary interfaces 8', 8" by means of a position adjustment of a single main body 5 supporting both motors 7', 7" and both rotary interfaces 8', 8" makes the rehabilitation device 1 particularly versatile and suitable for a high number of different motor gestures for each arm and combinations of motor gestures of both limbs, for example upper, synchronized, asynchronous, dependent, independent, symmetrical, non-symmetrical, in the same direction or in the opposite direction, simultaneous or in succession.

The possibilities of defining free movements adapted to the conditions of the individual patient are further increased by virtue of the set 12 of grip accessories 12.1, 12.2, ..., 12.n which are couplable to the rotary interfaces 8', 8".

**Detailed description of the connecting base 2**

According to an embodiment, the connecting base 2 can comprise a "U" or clamp shape defining a connecting seat 16 which is laterally accessible for receiving for example an edge of a table (support 4).

The connecting members 3 can comprise, for example, clamps and/or tightening screws and/or adhesive surfaces and/or suction cups and/or magnets and/or Velcro, and allow a stationary, but removable, reversible and repositionable connection of the connecting base 2 to the support 4.

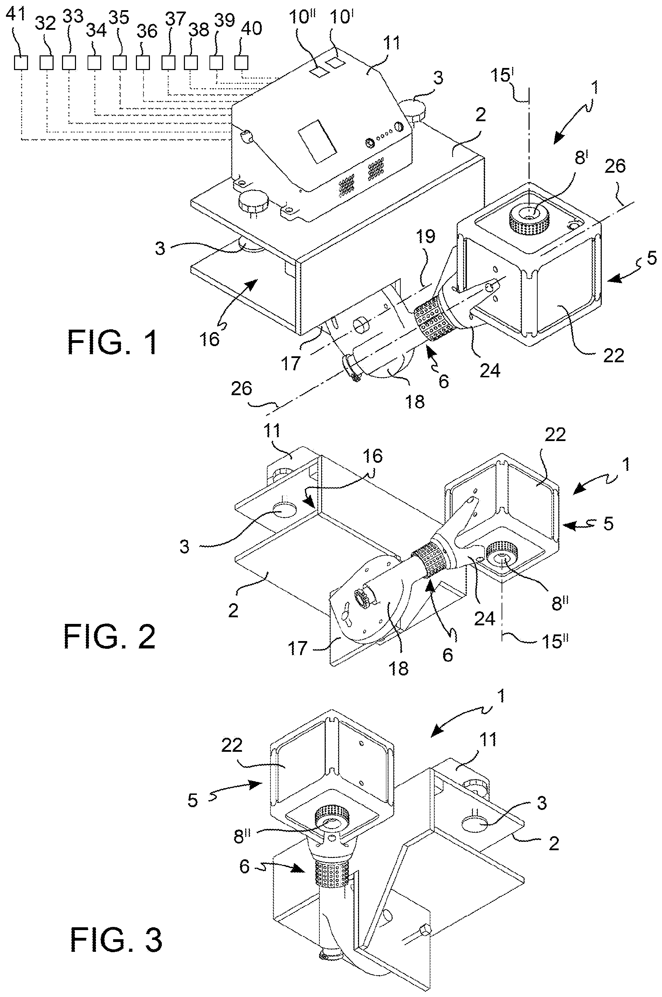

The connecting base 2 further forms a joint fastening portion 17 for fastening a base portion 18 of the adjustable joint 6 to the connecting base 2, for example for a fastening with the possibility of rotary adjustment of the position of the base portion 18 with respect to the connecting base 2, about an auxiliary adjustment axis 19, for example horizontal (figures 1, 5, 6).

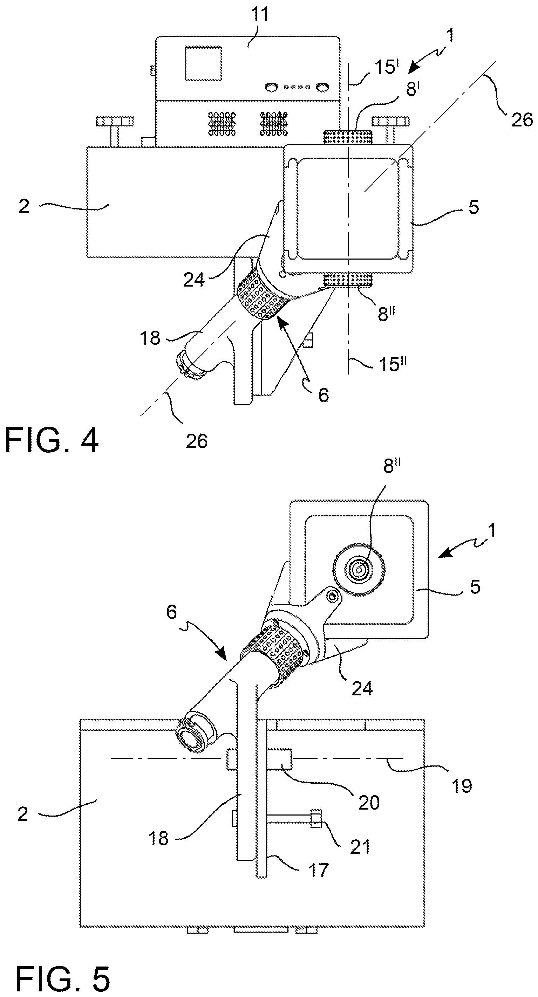

According to an embodiment, the base portion 18 and the joint fastening portion 17 can be connected to each other by means of a rotation pin 20 which constrains the adjustment movement thereof to rotation only about the auxiliary adjustment axis 19 and by means of a tightening bolt 21 inserted in a hole of one of the base portion 18 and the joint fastening portion 17, and in an arcuate groove of the other of the base portion 18 and the joint fastening portion 17 (figures 2, 5).

**Detailed description of the main body 5**

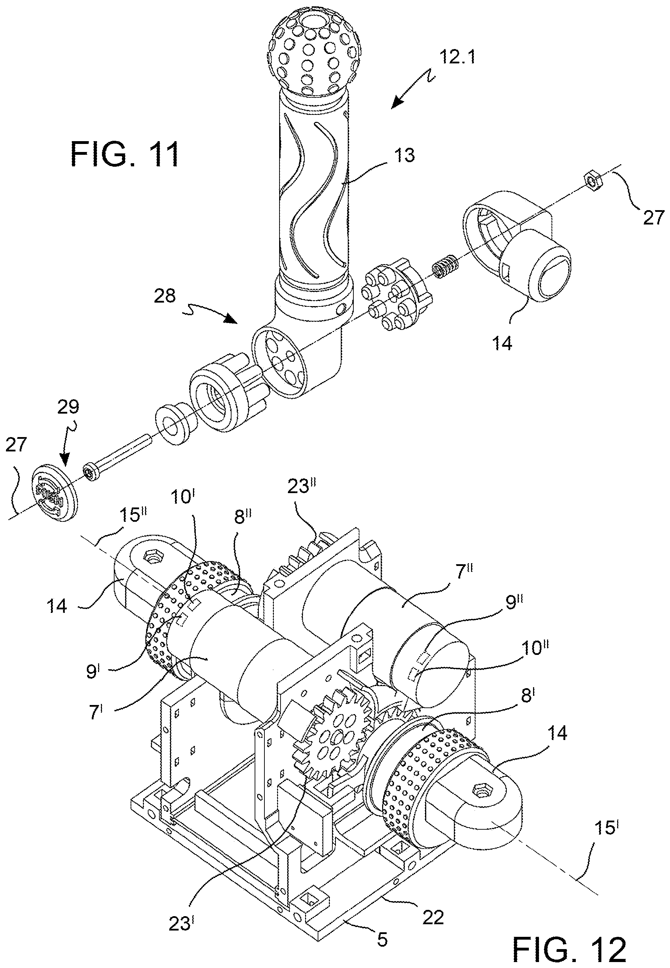

In accordance with an embodiment, the main body 5 has an outer housing 22, for example in the form of a parallelepiped or a cube or a sphere, which accommodates, at least partially but preferably completely, the first motor 7' and the second motor 7" therein and, if provided, a first transmission 23' (preferably with constant transmission ratio, preferably less than 1, i.e., a reduction ratio) connected between the first motor 7' and the first rotary interface 8', and a second transmission 23" (preferably with constant transmission ratio, preferably less than 1, i.e., a reduction ratio) connected between the second motor 7" and the second rotary interface 8" (Figure 12).

The first transmission 23' and/or the second transmission 23" can comprise, for example, a reduction gear or a belt and pulley transmission, for example reduction, or another similar and equivalent transmission system.

According to an embodiment, in order to reduce the overall dimensions of the main body 5 as much as possible, the first motor 7' and the second motor 7" are arranged parallel to each other and placed side by side, with the axial extensions thereof overlapping, so that the axial dimensions of both the first 7' and second 7" motors is smaller than the sum of the individual axial extensions thereof.

Still in order to reduce the overall dimensions of the main body 5 as much as possible, a first operating connection of the first motor 7' to the first rotary interface 8' (for example by means of the first transmission 23' or by means of "direct drive" connection) and a second operating connection of the second motor 7" to the second rotary interface 8" (for example by means of the second transmission 23" or by means of "direct drive" connection) are made/positioned on two opposite sides of the first 7' and second 7" motors or on two opposite sides of the main body 5. Preferably, the first transmission 23' is positioned laterally next to the second motor 7" with overlapping of the axial extensions thereof, and the second transmission 23" is positioned laterally next to the first motor 7' with overlapping of the axial extensions thereof, such that the total axial dimensions of both the first motor 7' + first transmission 21' and second motor 7" + second transmission 21" assemblies is identical to the individual axial dimensions of only one of the two first motor 7' + first transmission 21' and second motor 7" + second transmission 21" assemblies.

With further advantage, the first rotary interface 8' and the second rotary interface 8" are both in laterally displaced positions with respect to both the first motor 7' and the second motor 7". This avoids the accumulation of components one behind the other in the axial direction of the motors 7', 7", allowing a minimization of the dimensions of the main body 5 in the axial direction of the motors 7', 7" (Figures 12, 13).

In accordance with an embodiment, the first 8' and second 8" rotary interfaces are accessible from the outside of the main body 5 and preferably facing or turned towards two opposite directions and towards the outside of the main body 5.

Preferably, the first 8' and second 8" rotary interfaces are positioned on two opposite sides of the main body 5 and the first rotation axis 15' and the second rotation axis 15" are parallel, preferably coincident and coaxial.

The first 8' and second 8" rotary interfaces comprise quick coupling means, without the aid of tools, for example an elastic snap-fit coupler, a threaded ring nut coupler, a bayonet coupler, an insertion coupler or a threaded coupler, for a rotatably integral connection between the rotary interface 8', 8" and the coupling portion 14 of the grip accessory 12.1, 12.2, ..., 12.n.

This allows a quick and easy reconfiguration of the rehabilitation device 1, by replacing and modifying the grip accessories 12.1, ..., 12.n, even in the domestic environment and without the help of specialized personnel.

According to an embodiment, the adjustable joint 6 is configured so that the main body 5 is rotatable with respect to the connecting base 2 about a main adjustment axis 26 and lockable with respect to the connecting base 2, in which the main adjustment axis 26 is inclined with respect to the first rotation axis 15' and with respect to the second rotation axis 15".

The adjustable joint 6 can comprise an adjustable portion 24, integral with the main body 5, and rotatable with respect to the base portion 18 about a main adjustment axis 26 and lockable with respect to the base portion 18, in which the main adjustment axis 26 is inclined with respect to the first rotation axis 15' and with respect to the second rotation axis 15".

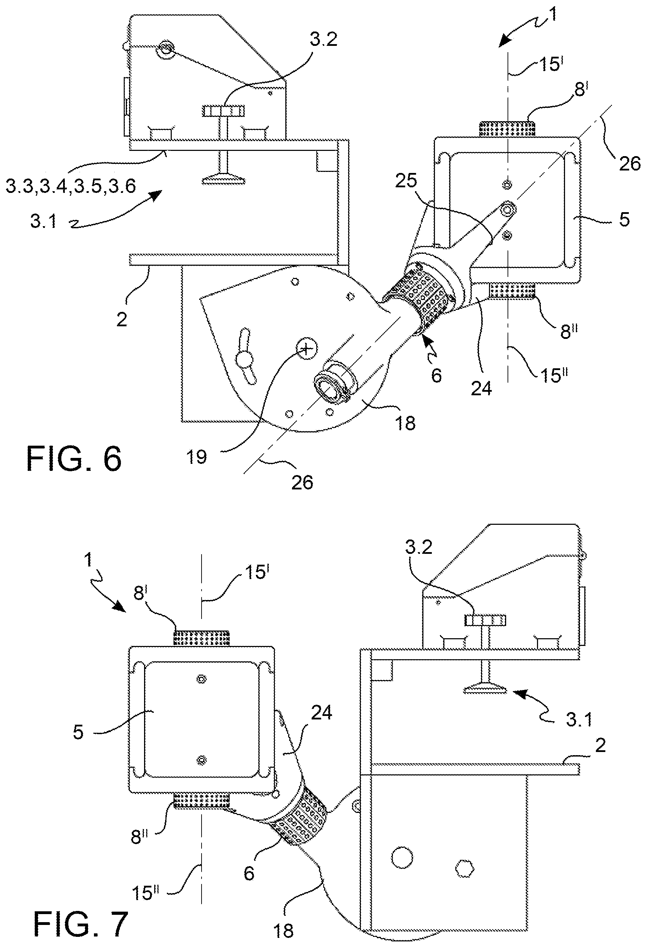

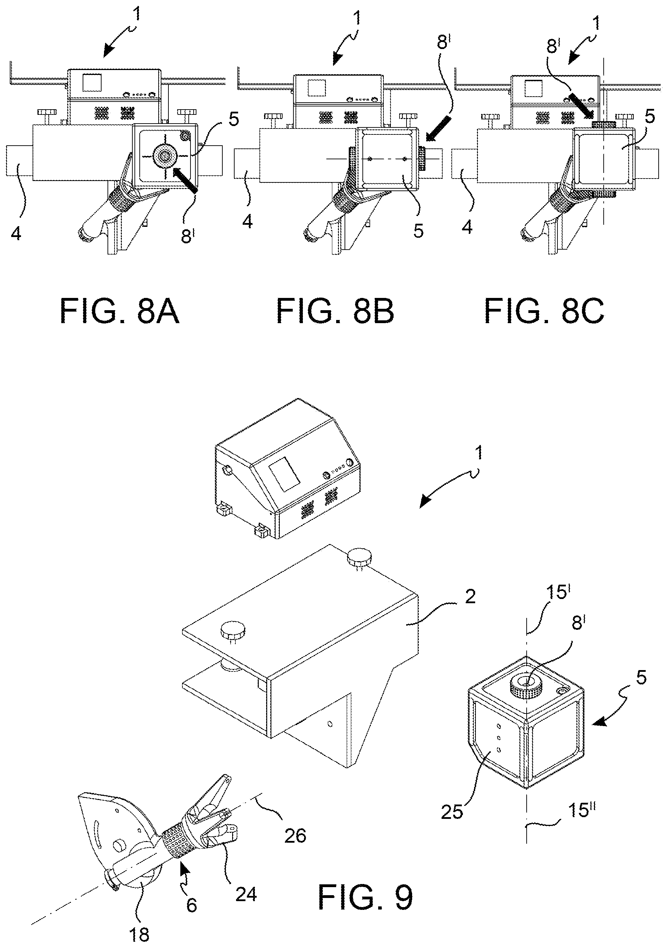

The adjustable portion 24 can be connected to the base body 5 in a fixed manner (not removable) or preferably, detachably (for example by screws) to a special support seat 25 of the main body 5 (Figures 6, 9). The detachable connection allows a storage and transport of the partially disassembled device 1 with particularly small dimensions and little packaging material.

By virtue of the inclination of the main adjustment axis with respect to the first rotation axis 15' and the second rotation axis 15", the latter can be oriented in the aforesaid at least three body positions in three mutually transverse, preferably orthogonal, directions.

More precisely, the main adjustment axis 26 is inclined with respect to (and preferably but not necessarily incident with) the rotation axes 15', 15" in turn coinciding with each other, at an angle in the range between 45° and 65°, preferably between 50° and 60°, more precisely at an angle arctan(Sqrt(2)), i.e., 54.74°. This allows the orientation of the first 15' and second 15" rotation axes in the space along mutually orthogonal directions (in Figures 8A, 8B, 8C the same rotary interface is indicated by an arrow to show the three orthogonal orientations).

For a cube shape of the main body 5 with the first rotation axis 15' and the second rotation axis 15" coinciding and centered on two opposite faces of the cube, the inclination angle between the main adjustment axis 26 or the first and second rotation axes 15', 15" is equal to the angle formed by the diagonal of the cube with the normal angle at each of its faces, equal to arctan(Sqrt(2)).

Detailed description of the set 12 of grip accessories 12.1, ..., 12.n

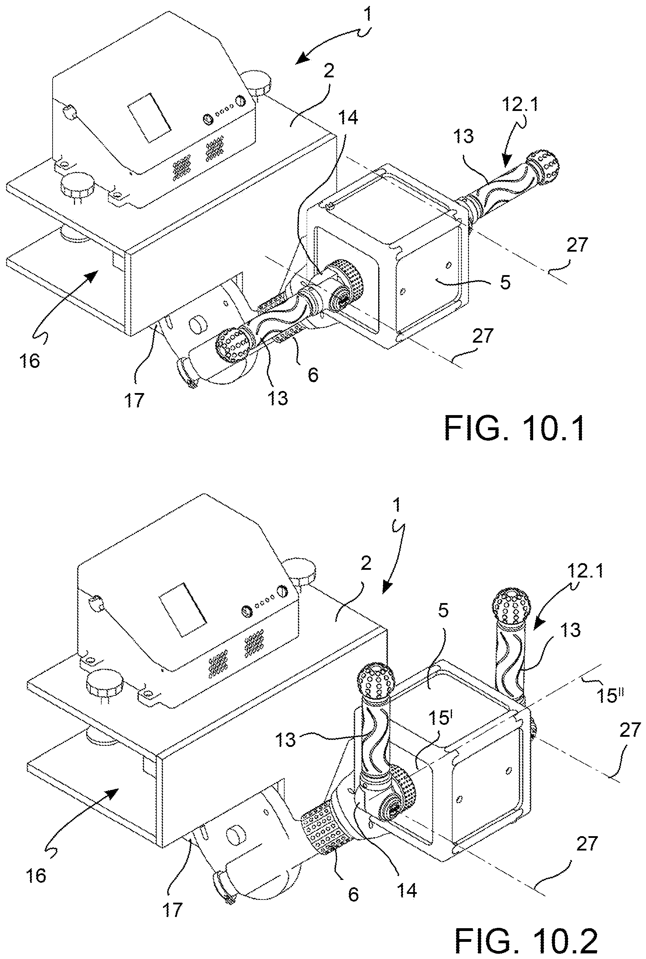

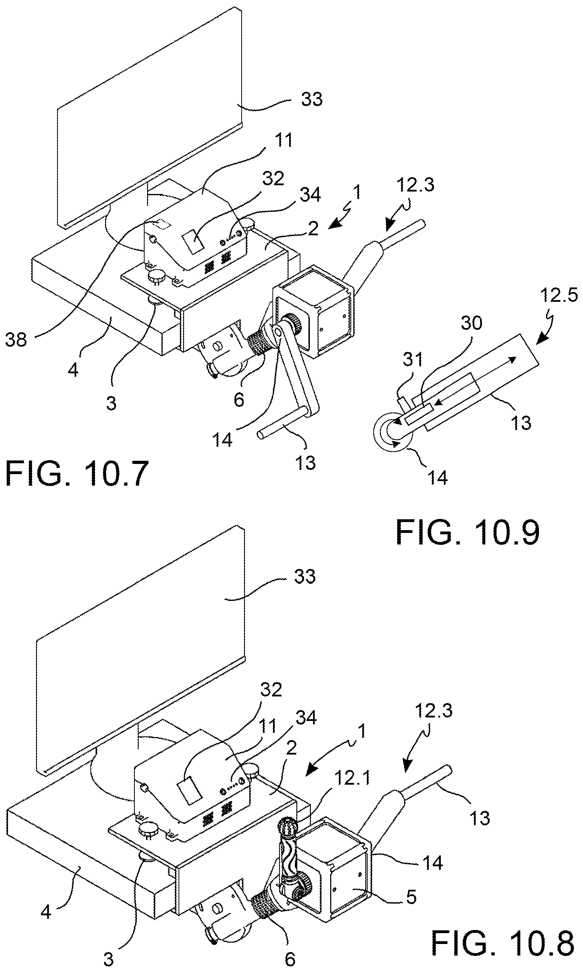

In accordance with an embodiment, the grip accessories 12.1, ..., 12.n comprise two handlebar accessories 12.1 (figures 10.1, 10.2) each couplable to one of the first 8' and second 8" rotary interfaces, respectively, in which the grip portion 13 is orientable (and lockable in position) with respect to the coupling portion 14, about a handlebar orientation axis 27 transverse (preferably orthogonal) to the first rotation axis 15' and to the second rotation axis 15".

To this end, the handlebar accessory 12.1 can comprise an orientation and locking mechanism 28 (figure 11), for example elastically lockable and releasable by pressing a button 29.

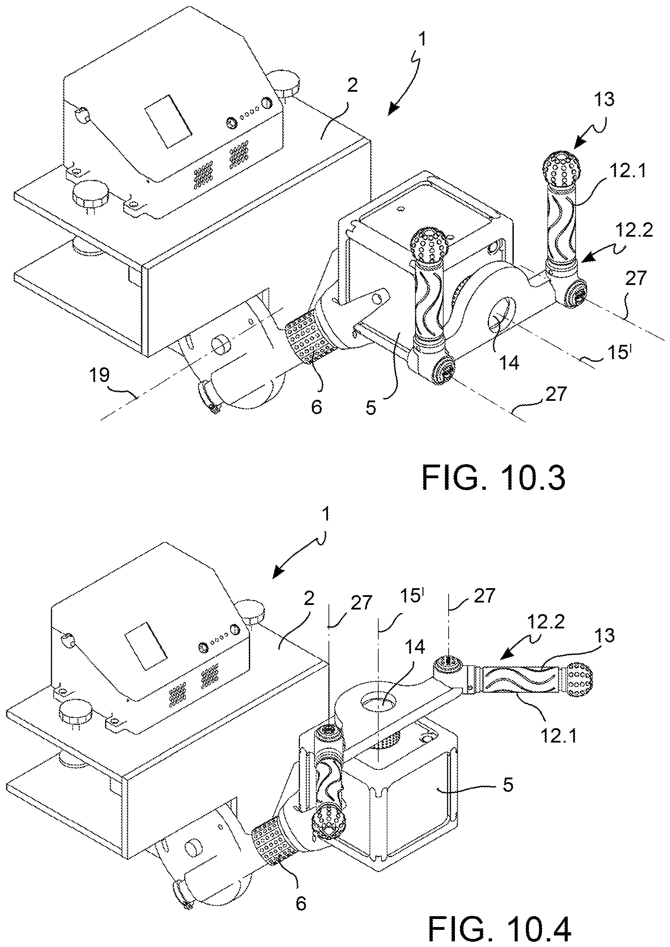

In accordance with an embodiment, the grip accessories 12.1, ..., 12.n comprise a wheel accessory 12.2 (figures 10.3, 10.4) couplable to one of the first 8' and second 8" rotary interfaces, and comprising two grip portions 13 spaced apart from each other and positioned on two opposite sides with respect to the first 8' or second 8" rotary interface, and adjustable (and lockable in position) with respect to the coupling portion 14, about respective handlebar orientation axes 27, 27 for example parallel to the first rotation axis 15' or to the second rotation axis 15".

To this end, the wheel accessory 12.2 can comprise for each grip portion 13 an orientation and locking mechanism 28 (figure 11), for example elastically lockable and releasable by pressing a button 29.

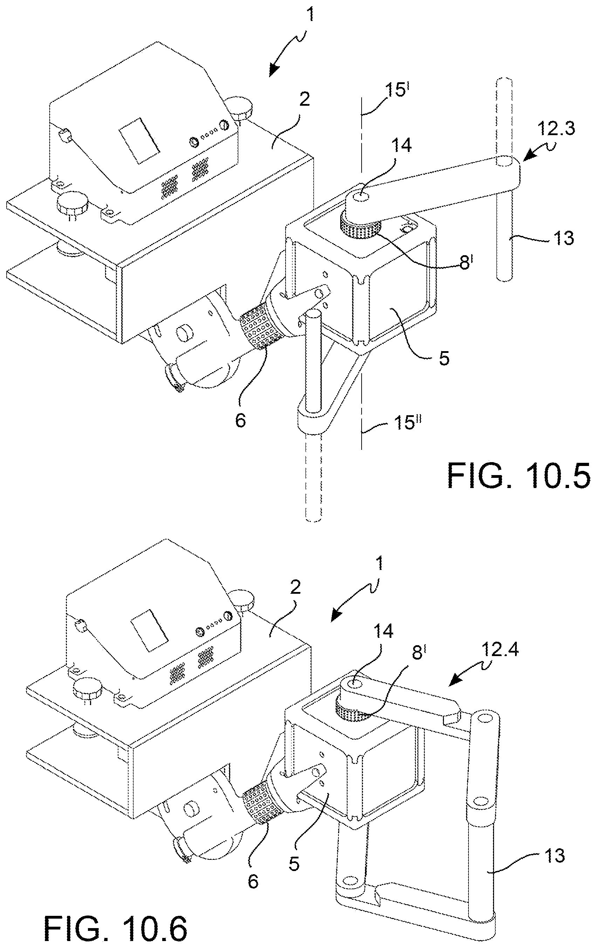

In accordance with an embodiment, the grip accessories 12.1, ..., 12.n comprise two crank accessories 12.3 (figure 10.5) each couplable to one of the first 8' and second 8" rotary interfaces, respectively, in which the grip portion 13 is parallel and spaced with respect to the first rotation axis 15' and the second rotation axis 15". The coupling portion 14 of the crank accessories 12.3 can be configured for coupling with the respective rotary interface 8', 8" in two different positions of the crank accessory 12.3, mutually overturned by 180° (Figure 10.5).

In accordance with an embodiment, the grip accessories 12.1, ..., 12.n comprise a linkage accessory 12.4 or articulated multilateral accessory (figure 10.6) having two coupling portions 14 both connected to a single grip portion 13 by means of two articulated linkages.

In accordance with an embodiment, the grip accessories 12.1, ..., 12.n comprise a translational accessory 12.5 (figure 10.9), in which the grip portion 13 is linearly translatable with respect to the coupling portion 14, and which comprises a non - self locking, for example rack, motion conversion mechanism 30 interposed between the grip portion 13 and the coupling portion 14, as well as anti-rotation means 31 which prevent a rotation of the grip portion 13 with respect to the main body 5.

Figure 10.8 shows an embodiment in which different grip accessories 12.1, ..., 12.n are connected to the main body 5.

The coupling portions 14 of the grip accessories 12.1, ..., 12.n have a sure angular reference for a connection with the rotary interface 8', 8" in a single angular position, allowing the electronic control device 11 to always know the spatial orientation thereof in a unique manner.

**Detailed description of the electronic control device 11**

The control device 11 can comprise a computer as shown in the figures, fastened for example on the connecting base 2, and provided with or connectable to a user interface 32 with a display 33 and/or a keyboard 34. Alternatively, the control device 11 can be inside the main body 5. In an embodiment, the control device 11 can be connected or connectable (by means of Wi-Fi, Bluetooth, etc.) to an external electronic device (e.g., a tablet computer or a smart phone) serving as a user interface.

The control device 11 is configured to allow the selection of a plurality of rehabilitation programs/modes with the possibility of adjusting a plurality of movement parameters of the respective rehabilitation program/mode.

The rehabilitation programs/modes comprise rehabilitation programs with synchronized movements of the first rotary interface 8' and the second rotary interface 8" and rehabilitation programs with movements of the first rotary interface 8' and the second rotary interface 8" which are mutually different.

In accordance with an embodiment, the control device 11 is configured to perform rehabilitation programs with a control of the first motor 7' and the second motor 7" by means of a control in position and in rotary speed, particularly suitable for passive rehabilitation, in which the rehabilitation device 1 imposes a predefined movement on the user's limbs.

In accordance with an additional or alternative embodiment, the control device 11 is (further) configured to execute rehabilitation programs with a control of the first motor 7' and the second motor 7" by means of an impedance and/or input control, based on:

- movements/positions of the first rotary interface 8' and of the second rotary interface 8" detected by the first position detecting means 9' and second position detecting means 9",
- theoretical movements/positions of the first rotary interface 8' and of the second rotary interface 8" commanded by the control device 11, and
- the corresponding torques applied/transmitted to the first rotary interface 8' and to the second rotary interface 8", detected by the first torque detecting means 10' and second torque detecting means 10".

The impedance/admittance control is particularly suitable for a motor rehabilitation in which movements carried out by the user themselves are supported to the extent necessary ("assist-as-needed") so as to support the (residual) motor skills of the user based on his real residual abilities at the time of use of the device 1.

According to an embodiment, the control device 11 is configured or configurable for a control of the movement of the first motor 7' and the second motor 7" in a coordinated and dependent manner, according to one or more dependency parameters and/or one or more dependency functions, for example predefined or selectable by the user interface 32.

The dependency parameters or functions of the movements of the first motor 7' and of the second motor 7" can be selectable so that the second motor 7" is controlled depending on a detected movement of the first rotary interface 8', so as to impose a movement of the second rotary interface 8":

- in time offset with respect to the movement of the first rotary interface 8' by a time offset value (selectable as a function of rehabilitation needs), and/or
- in position offset, forward or backward, with respect to the movement of the first rotary interface 8' by a position offset value (selectable as a function of rehabilitation needs), and/or
- in the opposite direction with respect to the movement of the first rotary interface 8' with or without time offset and/or position offset.

In accordance with an embodiment, the first and second position detecting means 9', 9" comprise an encoder for each of the first 8' and second 8" rotary interfaces (such encoders can preferably be placed directly on the first 7' and second 7" motors), but possibly no further sensor for the direct measurement of rotational speeds, or alternatively a first rotary speed sensor for detecting the rotary speed of the first rotary interface 8' and a second rotary speed sensor for detecting the rotary speed of the second rotary interface 8" could be provided.

The angular position detection can be redundant (two encoders or angular position detectors associated with each of the two rotary interfaces 8', 8") with consistency check, with angular position detection incrementally and/or absolutely, so as to avoid a homing procedure (initial reset of the starting angular position). The rotational speed value can be estimated by the control device 1, using a closed-loop logic, as depicted in Figure 14.1. The angular position measure $\theta_m$ of each rotary interface 8', 8" is compared with the estimated angular position value $\theta_s$ starting from the current rotational speed estimate $\dot{\theta}_s$. The calculated error $\Delta\theta$ is then used within a PID logic (Proportional-Integrative-Derivative, or similar processing module) which outputs the rotational speed value estimate for the next control cycle.

In accordance with an embodiment, using the speed estimation method described above, the control device 11 can be configured to implement a second closed loop for controlling the first rotary interface 8' and the second rotary interface 8" in speed as shown in Figure 14.2. The error between the reference rotational speed $\dot{\theta}_r$ and the actually estimated rotational speed $\theta_s$ starting from the measured position $\theta_m$ is used within a PID logic (or analogous processing module) which outputs the motor command $U_c$ for the motor 7', 7" for the corresponding rotary interface 8', 8".

In order to ensure the safety of the user, the control device 11 continuously monitors the current absorbed by the motors 7', 7" and the rotational speeds and torques transmitted by means of the first 8' and second 8" rotary interfaces, verifying that appropriate imposed limits are not exceeded.

According to an embodiment, the first torque detecting means 10' and the second torque detecting means 10" can comprise means for detecting the current absorbed by the first motor 7' and the second motor 7", respectively, and means for estimating/determining the transmitted torque as a function of the detected absorbed current.

According to an embodiment, the first torque detecting means 10' and the second torque detecting means 10" can comprise one or more load cells, for example connected to the motor 7', 7" or to the rotary interface 8', 8", and configured to provide a signal representative of the torque transmitted respectively to the/from the first rotary interface 8' and/or the second rotary interface 8".

Similarly, the closed loop for controlling each of the first 8' and second 8" rotary interfaces in position is shown in Figure 14.3. The error between the reference angular position $\theta_r$ and the currently measured angular position $\theta_m$ is used within a PID logic (or a processing/adjustment module other than PID) to generate a speed command $\dot{\theta}_c$ for the motor 7', 7". The new rotational speed reference is then input to the rotational speed control cycle previously described (Figure 14.2) to generate the actual motor command $U_c$.

According to an embodiment, the control device 11 is configured to monitor the tracking error (i.e., the difference between the position value and/or actual angular speed, detected, and the commanded target value thereof) to implement a fail safe operation.

According to an embodiment, with the aim of controlling both the angular position and the moment (torque) transmitted by each of the first 8' and second 8" rotary interfaces, it is advantageous to implement by means of the control device 11 (all the method algorithms described herein are advantageously executed by the control device 11 which is thus configured to execute them) an admittance control loop, shown for example in figure 14.4. The control device 11 makes an estimate of the torque $C_m$ exerted by each of the first 7' and second 7" motors (for example proportional to the absorbed current $I_a$ by means of the proportionality coefficient $K_t$) and compares the estimated torque value $C_m$ with the reference torque $C_r$. The calculated difference in torque $\Delta C$ between the estimated torque and the reference torque is applied ("made to act") on a virtual model with predefined admittance features. By virtue of this approach, it is possible to define a reference rotary speed $\dot{\theta}_c$ which is then input to the speed control previously described (Figure 14.2) to obtain the actual command $U_c$ at the first 8' and second 8" rotary interfaces, i.e., at the grip accessories 12.1, ..., 12.n where a torque $C_u$ is exchanged with the user.

According to an embodiment, one or more or all of the described control patterns are applied to each of the first 8' and second 8" rotary interfaces of the device 1 in an independent manner.

Alternatively or (advantageously) additionally, the control device 11 is configured or settable by means of program selection by a user, so as to control the movements and the torques applied to the first 8' and second 8" rotary interfaces in a dependent manner, in order to obtain a bilateral (bi-limb) device-user interaction.

According to an embodiment, the movements and the torques applied to the first 8' and second 8" rotary interfaces, or in other words the first 8' and second 8" rotary interfaces, are (dynamically) linked to each other by the logic (dynamic model of motion and torque coupling) diagrammatically depicted in Figure 14.5. In Figure 14.5, $p_1$ and $p_2$ indicate the two first 8' and second 8" rotary interfaces, while $t$ represents a virtual transmission element capable of scaling the effect of the two first 8' and second 8" rotary interfaces by means of the scale parameters $\tau_1$ and $\tau_2$. As depicted, $p_1$ and $p_2$ are linked to $t$ through virtual impedances characterized by stiffness and damping values respectively indicated with $k_1$, $d_1$ and $k_2$, $d_2$ and having preset, selectable and/or adjustable values. In turn, the virtual transmission element $t$ is connected to the absolute reference $xy$ by means of a spring-damper system (numerical model) characterized by the stiffness and damping values indicated with $k_t$, $d_t$ and having preset, selectable and/or adjustable values. The angles formed by $p_1$, $p_2$ and $t$ with the absolute reference system $xy$ are indicated by $\theta_1$, $\theta_2$ and $\alpha$ re-

spectively. Furthermore, $\varphi_1$ and $\varphi_2$ represent the relative angle measured between $p_1$, $t$ and $p_2$, $t$, respectively. The torques acting on $p_1$ and $p_2$ are indicated by $C_1$ and $C_2$, respectively, while $C_t$ represents an external torque applied directly to $t$ to assist the user's movement. Finally, $C_{u1}$ and $C_{u2}$ are the torques transmitted to the first 8' and second 8" rotary interfaces by the user's limbs.

By virtue of this approach, by modifying the described control parameters, it is possible both to adjust the torque intensity applied from the outside and the torque intensity which one limb is capable of transmitting to the other, and to scale the ranges of movement of the two limbs based on the user's residual mobility.

By exploiting and applying the method and dynamic model of bilateral dependence (bimanual or bi-foot) described, according to an embodiment the control device 11 is configured to implement a control method shown by way of example in figure 14.6.

For each of the first 8' and second 8" rotary interfaces, the control device calculates angular position and rotary speed based on the absolute angular position measurement as follows.

$$\varphi_1 = \theta_1 - \alpha$$

$$\varphi_2 = \theta_2 - \alpha$$

Depending on the rotary speed values, the control device 11 calculates the torques applied on the virtual impedances and then transmitted on the virtual transmission element.

$$C_1 = k_1 \varphi_1 + d_1 \dot{\varphi}_1$$

$$C_2 = k_2 \varphi_2 + d_2 \dot{\varphi}_2$$

Depending on the positions and angular speeds, as well as the torques applied on the virtual transmission element, the control device 11 solves the dynamic motion equations of the virtual transmission to extrapolate the measurements of $\alpha$ and $\dot{\alpha}$ (angular position and angular speed) for the next control cycle.

$$k_t \alpha + d_t \dot{\alpha} = \tau_1 C_1 + \tau_2 C_2 + C_t$$

The angular position and angular speed values (here synonymous with rotary speed) of the extrapolated virtual transmission element are used by the control device 11 as a basis for generating the actual power supply command $U_c$ sent to each of the first 7' and second 7" motors.

In accordance with embodiments, the control device 11 is in signal communication with one or more of:

- one or more electromyographic sensors 35,
- one or more functional electrical stimulation devices 36,
- one or more EEG signal detectors 37,
- one or more electronic graphical and/or auditory user interfaces 38,
- one or more EDA (electrodermal activity) sensors 39,
- one or more ECG (electrocardiac activity) sensors 40,
- one or more digital cameras 41 for monitoring the user and/or movements of the user's limbs,

and is configured to control the first motor 7' and the second motor 7" depending on signals provided thereby.

According to an embodiment, the first 8' and second 8" rotary interfaces comprise electrical contact means for transmitting electrical signals between the grip accessory 12.1, ..., 12.n and the control device 11, for example for transmitting signals generated by one or more force or pressure sensors connected to the grip portion 13, and/or for transmitting activation signals for one or more light signals (LEDs) connected to the grip portion 13.

In the described examples the motors 7', 7" are electric motors, but they can also be different motors, for example pneumatic motors.

The rehabilitation device 1 allows carrying out different motor gestures in bilateral rehabilitation activities, capable of involving the joints of the limb, for example upper, in a different manner. This is possible by virtue of the possibility of using different grip accessories 12.1, ..., 12.n, in combination with the possibility of versatile orientation of the main body 5 and the relative rotation axes 15', 15".

The technical reference field is that related to neuromotor rehabilitation, in which the availability of devices capable of supporting the patient during the neuromotor recovery phases is of considerable utility. Even further, considering the portability of the device 1, it lends itself well to also being used in contexts outside the hospital and in tele-rehabilitation, allowing an autonomous use by the patient.

Obviously, in order to meet contingent and specific needs, those skilled in the art may make further changes and variations to the rehabilitation device 1 according to the present invention, all falling within the scope of protection of the invention, as defined by the following claims.

**Reference numerals**

1 rehabilitation device
2 connecting base
3 connecting members
3.1 clamp
3.2 tightening screw

3.3 adhesive surface
3.4 suction cup
3.5 magnet
3.6 Velcro
4 support
5 main body
6 adjustable joint
7', 7" first motor, second motor
8', 8" first rotary interface, second rotary interface
9', 9" first position detecting means, second position detecting means,
10', 10" first torque detecting means, second torque detecting means,
11 electronic control device
12 set of grip accessories
12.1 handlebar accessory
12.2 wheel accessory
12.3 crank accessory
12.4 linkage accessory
12.5 translational accessory
13 grip portion
14 coupling portion
15', 15" first, second rotation axis
16 connecting seat
17 joint fastening portion
18 base portion of the adjustable joint
19 auxiliary adjustment axis
20 rotation pin
21 tightening bolt
22 outer housing
23', 23" first, second transmission
24 adjustable portion of the adjustable joint
25 support seat of the main body
26 main adjustment axis
27 handlebar orientation axis
28 orientation and locking mechanism
29 button
30 motion conversion mechanism
31 anti-rotation means
32 user interface
33 display
34 keyboard
35 electromyographic sensor
36 functional electrical stimulation device
37 EEG signal detector
38 auditory user interface,
39 EDA (electrodermal activity) sensor
40 ECG (electrocardiac activity) sensors
41 digital camera

## Claims

1. A device (1) for the bilateral rehabilitation of a user's limbs, comprising:

   - a connecting base (2) for a stationary connection of the connecting base (2) to a support (4),

   - a main body (5) connected with the connecting base (2),
   - a first motor (7') and a second motor (7") supported by the main body (5),
   - a first rotary interface (8') supported by the main body (5) in a first position and which is rotatably operable about a first rotation axis (15') by the first motor (7'), and
   - a second rotary interface (8") supported by the main body (5) in a second position spaced from the first position and which is rotatably operable about a second rotation axis (15") by the second motor (7"),
   - a set (12) of grip accessories (12.1, 12.2, ..., 12.n) each having a grip portion (13) and a coupling portion (14) which are reversibly couplable and integral in rotation to one of the first (8') and second (8") rotary interfaces, respectively,
   - an electronic control device (11) in signal connection with the first motor (7') and with the second motor (7") and comprising:

     - first position detecting means (9') for detecting an angular position of the first rotary interface (8') with respect to the main body (5),
     - second position detecting means (9") for detecting an angular position of the second rotary interface (8") with respect to the main body (5),

   wherein the electronic control device (11) is configured to control the rotary movements applied to the first (8') and second (8") rotary interfaces depending on the angular positions detected by the first and second position detection means (9', 9"),
   **characterised in that**
   the main body (5) is connected with the connecting base (2) by an adjustable and lockable joint (6) which allows an adjustment and locking of the position of the main body (5) with respect to the connecting base (2) in at least three different body positions with different orientations of the first (15') and second (15") rotation axes.

2. A device (1) according to claim 1, wherein the electronic control device (11) further comprises:

   - first torque detecting means (10') for detecting a torque applied to the first rotary interface (8'),
   - second torque detecting means (10") for detecting a torque applied to the second rotary interface (8"),

   wherein the electronic control device (11) is configured to control the rotary movements and torques applied to the first (8') and second (8") rotary interfaces depending on the angular positions detected by the first and second position detecting means (9', 9") and/or the torques detected by the first and second torque detecting means (10', 10").

3. A device (1) according to any one of the preceding claims, wherein the connecting base (2) comprises a "U" or clamp shape defining a connecting seat (16) which is laterally accessible to be able to receive an edge of a table.

4. A device (1) according to any one of the preceding claims, wherein the connecting base (2) forms a joint fastening portion (17) for fastening a base portion (18) of the adjustable joint (6) to the connecting base (2), with the possibility of rotary adjustment of the base position (18) with respect to the connecting base (2) about a horizontal auxiliary adjustment axis (19).

5. A device (1) according to any one of the preceding claims, wherein the main body (5) comprises an outer housing (22) which accommodates the first motor (7') and the second motor (7"), in which:

   - the first motor (7') and the second motor (7") are arranged parallel to each other and side by side, with the axial extensions thereof overlapping, so that the axial dimension of both the first (7') and second (7") motors is less than the sum of the individual axial extensions thereof,
   - a first operating connection of the first motor (7') to the first rotary interface (8') and a second operating connection of the second motor (7") to the second rotary interface (8") are positioned on opposite sides of the first (7') and second (7") motors, and
   - the first operating connection (23') is positioned laterally next to the second motor (7") with the axial extensions thereof overlapping, and the second operating connection (23") is positioned laterally next to the first motor (7") with the axial extensions thereof overlapping,
   - the first rotary interface (8') and the second rotary interface (8") are both in laterally displaced positions with respect to both the first motor (7') and the second motor (7").

6. A device (1) according to any one of the preceding claims, wherein the first (8') and second (8") rotary interfaces are accessible from the outside of the main body (5) and facing two opposite directions towards the outside of the main body (5).

7. A device (1) according to any one of the preceding claims, wherein the first (8') and second (8") rotary interfaces are positioned on two opposite sides of the main body (5) and the first rotation axis (15') and the second rotation axis (15") are parallel or coincident.

8. A device (1) according to any one of the preceding claims, wherein the first (8') and second (8") rotary interfaces comprise quick coupling means, without the aid of tools, or one of:

   - an elastic snap-fit coupler,
   - a threaded ring nut coupler,
   - a bayonet coupler,
   - an insertion coupler,
   - a threaded coupler,

   for a rotationally locked connection between the rotary interface (8', 8") and the coupling portion (14) of the grip accessory (12.1, 12.2, ..., 12.n).

9. A device (1) according to any one of the preceding claims, wherein the adjustable joint (6) is configured so that the main body (5) is rotatable with respect to the connecting base (2) about a main adjustment axis (26) and lockable with respect to the connecting base (2),
   wherein the main adjustment axis (26) is inclined with respect to the first rotation axis (15') and with respect to the second rotation axis (15").

10. A device (1) according to claim 9, wherein the main adjustment axis (26) is inclined with respect to the rotation axes (15', 15") at an angle between 45° and 65°, or between 50° and 60°, or 54.7°, so as to allow, by means of a rotary adjustment about only the main adjustment axis (26), the orientation of the first (15') and second (15") rotation axes in the space along three directions orthogonal to each other.

11. A device (1) according to any one of the preceding claims, wherein the grip accessories (12.1,..., 12.n) comprise two handlebar accessories (12.1) each able to couple to one of the first (8') and second (8") rotary interfaces, respectively, wherein the grip portion (13) is orientable and lockable in position with respect to the coupling portion (14), about a handlebar orientation axis (27) orthogonal to the first rotation axis (15') and the second rotation axis (15") with the handlebar accessory (12.1) coupled to the rotary interface (8', 8").

12. A device (1) according to any one of the preceding claims, wherein the grip accessories (12.1, ..., 12.n) comprise a wheel accessory (12.2) able to couple to one of the first (8') and second (8") rotary interfaces and having two grip portions (13) spaced apart and positioned on two opposite sides with respect to the first (8') or second (8") rotary interface, and adjustable and lockable in position with respect to the coupling portion (14), around respective handlebar orientation axes (27, 27).

13. A device (1) according to any one of the preceding claims, wherein the grip accessories (12.1,..., 12.n) comprise two crank accessories (12.3) each coupled respectively to one of the first (8') and second (8") rotary interfaces, wherein the grip portion (13) is parallel and spaced with respect to the first rotation axis (15') and with respect to the second rotation axis (15").

14. A device (1) according to any one of the preceding claims, wherein the grip accessories (12.1,..., 12.n) comprise a linkage accessory (12.4) having two said coupling portions (14) both connected to a single grip portion (13) by means of two articulated linkages.

15. A device (1) according to any one of the preceding claims, wherein the grip accessories (12.1, ..., 12.n) comprise a translational accessory (12.5) the grip portion (13) of which is linearly translatable with respect to the coupling portion (14), and which comprises a non-automatically locking, for example rack, motion conversion mechanism (30) interposed between the grip portion (13) and the coupling portion (14), as well as anti-rotation means (31) which prevent a rotation of the grip portion (13) with respect to the main body (5).

16. A device (1) according to any one of the preceding claims, wherein the coupling portions (14) of the grip accessories (12.1, ..., 12.n) form a sure angular reference for a connection with the rotary interface (8', 8") in a single relative angular position.

17. A device (1) according to any one of the preceding claims, wherein the control device (11):

   - comprises a computer connectable to a user interface (32) with a display (33),
   - is configured to allow the selection of a plurality of rehabilitation programs with the possibility of adjusting a plurality of movement parameters of the respective rehabilitation program,

   wherein the rehabilitation programs comprise rehabilitation programs with movements of the first rotary interface (8') and the second rotary interface (8") which are not synchronized with each other.

18. A device (1) according to any one of the preceding claims, wherein the control device (11) is configured to perform:

   - rehabilitation programs with a control of the first motor (7') and the second motor (7") by means of an angular position control,
   - rehabilitation programs with a control of the first motor (7') and the second motor (7") by means of a rotary speed control.

19. A device (1) according to any one of the preceding claims, wherein the control device (11) is configured to perform rehabilitation programs with a control of the first motor (7') and the second motor (7") by means of an impedance and/or admittance control, based on:

   - angular positions of the first rotary interface (8') and of the second rotary interface (8") detected by the first position detecting means (9') and second position detecting means (9"),
   - theoretical angular positions of the first rotary interface (8') and of the second rotary interface (8") controlled by the control device (11), and
   - the corresponding torques transmitted to the first rotary interface (8') and to the second rotary interface (8"), detected by the first torque detecting means (10') and second torque detecting means (10").

20. A device (1) according to any one of the preceding claims, wherein the control device (11) is configured for a control of the movement of the first motor (7') and to the second motor (7") in a dependent manner, as a function of one or more dependency parameters and dependency functions, which are predefined or selectable by a user interface (32).

21. A device (1) according to any one of the preceding claims, wherein the control device (11) is in signal communication with one or more of:

   - one or more electromyographic sensors (35),
   - one or more functional electrical stimulation devices (36),
   - one or more EEG signal detectors (37),
   - one or more electronic graphical (33) and/or auditory (38) user interfaces,
   - one or more EDA sensors (39),
   - one or more ECG sensors (40),
   - one or more digital cameras (41),

   and is configured to control the first motor (7') and the second motor (7") depending on signals provided thereby.

22. A device (1) according to any one of the preceding claims, wherein both the first (15') and second (15") rotation axes have a fixed relative position therebetween.

**Patentansprüche**

1. Vorrichtung (1) zur bilateralen Rehabilitation der Gliedmaßen eines Benutzers, umfassend:

- eine Verbindungsbasis (2) zu der festen Verbindung der Verbindungsbasis (2) mit einer Stütze (4),
- einen Hauptkörper (5), der mit der Verbindungsbasis (2) verbunden ist,
- einen ersten Motor (7') und einen zweiten Motor (7"), die von dem Hauptkörper (5) getragen werden,
- eine erste Drehschnittstelle (8'), die von dem Hauptkörper (5) in einer ersten Position getragen wird und um eine erste Drehachse (15') durch den ersten Motor (7') drehbar betreibbar ist, und
- eine zweite Drehschnittstelle (8"), die von dem Hauptkörper (5) in einer zweiten, Position getragen wird, die von der ersten Position beabstandet ist und die um eine zweite Drehachse (15") durch den zweiten Motor (7") drehbar betreibbar ist,
- einen Satz (12) von Griffzubehörteilen (12.1, 12.2, ... , 12.n), die jeweils einen Griffabschnitt (13) und einen Kupplungsabschnitt (14) aufweisen, die reversibel kuppelbar sind und vollständig mit einer der ersten (8') und der zweiten (8") Drehschnittstellen drehbar sind,
- eine elektronische Steuervorrichtung (11), die in Signalverbindung mit dem ersten Motor (7') und dem zweiten Motor (7") steht und umfassend:
- erste Positionserkennungsmittel (9') zur Erkennung einer Winkelposition der ersten Drehschnittstelle (8') in Bezug auf den Hauptkörper (5),
- zweite Positionserkennungsmittel (9") zur Erkennung einer Winkelposition der zweiten Drehschnittstelle (8") in Bezug auf den Hauptkörper (5),

wobei die elektronische Steuervorrichtung (11) dazu konfiguriert ist, die Drehbewegung zu steuern, die auf die erste (8') und die zweite (8") Drehschnittstellen in Abhängigkeit von den Winkelpositionen, die durch die ersten und zweiten Positionsdetektionsmittel (9', 9") erkannt werden, angewendet wird, **dadurch gekennzeichnet, dass**
der Hauptkörper (5) mit der Verbindungsbasis (2) durch eine verstellbare und verriegelbare Kupplung (6) verbunden ist, die eine Einstellung und Verriegelung der Position des Hauptkörpers (5) in Bezug auf die Verbindungsbasis (2) in mindestens drei verschiedenen Körperpositionen mit verschiedenen Orientierungen der ersten (15') und der zweiten (15") Drehachsen ermöglicht.

2. Vorrichtung (1) nach Anspruch 1, wobei die elektronische Steuervorrichtung (11) ferner umfasst:

- erste Drehmomenterkennungsmittel (10') zur Erkennung eines Drehmoment, das auf die erste Drehschnittstelle (8') angewendet wird,
- zweite Drehmomenterkennungsmittel (10") zur Erkennung eines Drehmoments, das auf die zweite Drehschnittstelle (8") angewendet wird,

wobei die elektronische Steuervorrichtung (11) dazu konfiguriert ist, die Drehbewegungen und Drehmomente zu steuern, die auf die erste (8') und die zweite (8") Drehschnittstellen in Abhängigkeit von den Winkelpositionen, die durch die ersten und die zweiten Positionserkennungsmittel (9', 9") erkannt werden, und/oder den Drehmomenten, die durch die ersten und die zweiten Drehmomenterkennungsmittel (10', 10") erkannt werden, angewendet werden.

3. Vorrichtung (1) nach einem der vorhergehenden Ansprüche, wobei die Verbindungsbasis (2) eine U- oder Klemmgestalt aufweist, die einen Verbindungssitz (16) definiert, die seitlich zugänglich ist, um einen Rand eines Tisches aufnehmen zu können.

4. Vorrichtung (1) nach einem der vorhergehenden Ansprüche, wobei die Verbindungsbasis (2) einen Kupplungsbefestigungsabschnitt (17) ausbildet, um einen Basisabschnitt (18) der verstellbaren Kupplung (6) an der Verbindungsbasis (2) zu befestigen, mit der Möglichkeit einer Drehverstellung der Basisposition (18) in Bezug auf die Verbindungsbasis (2) um eine horizontale Hilfsverstellungsachse (19).

5. Vorrichtung (1) nach einem der vorhergehenden Ansprüche, wobei der Hauptkörper (5) ein Außengehäuse (22) umfasst, das den ersten Motor (7') und den zweiten Motor (7") aufnimmt, wobei:

- der erste Motor (7') und der zweite Motor (7") parallel zueinander und nebeneinander angeordnet sind, mit deren axialen Erstreckungen, die sich überlappen, sodass die axiale Abmessung sowohl des ersten (7') als auch des zweiten (7") Motors kleiner als die Summe ihrer einzelnen axialen Erstreckungen ist,
- eine erste Betriebsverbindung des ersten Motors (7') mit der ersten Drehschnittstelle (8') und eine zweite Betriebsverbindung des zweiten Motors (7") mit der zweiten Drehschnittstelle (8") auf gegenüberliegenden Seiten der ersten (7') und zweiten (7") Motoren positioniert sind, und
- die erste Betriebsverbindung (23') seitlich neben dem zweiten Motor (7") mit ihren axialen Erstreckungen, die sich überlappen, positioniert ist und die zweite Betriebsverbindung (23") seitlich neben dem ersten Motor (7') mit ihren axialen Erstreckungen, die sich überlappen, positioniert ist,

- sich beide die erste Drehschnittstelle (8') und die zweite Drehschnittstelle (8") in seitlich versetzten Positionen in Bezug auf sowohl den ersten Motor (7') als auch den zweiten Motor (7") befinden.

6. Vorrichtung (1) nach einem der vorhergehenden Ansprüche, wobei die erste (8') und zweite (8") Drehschnittstellen von außen des Hauptkörpers (5) zugänglich sind und zwei gegenüberliegenden Richtungen nach außen des Hauptkörpers (5) zugewandt sind.

7. Vorrichtung (1) nach einem der vorhergehenden Ansprüche, wobei die erste (8') und die zweite (8") Drehschnittstellen an zwei gegenüberliegenden Seiten des Hauptkörpers (5) positioniert sind und die erste Drehachse (15') und die zweite Drehachse (15") parallel oder zusammenpassend sind.

8. Vorrichtung (1) nach einem der vorhergehenden Ansprüche, wobei die erste (8') und zweite (8") Drehschnittstellen Schnellkupplungsmittel, ohne Hilfe von Werkzeugen, oder eines der folgenden Elemente aufweisen:

   - eine elastische Schnappverbindung-Koppler,
   - einen Ring-Nut Gewindekoppler,
   - einen Bajonettkoppler,
   - einen Einführungskoppler,
   - einen Gewindekoppler,

   für eine drehfeste Verbindung zwischen der Drehschnittstelle (8', 8") und dem Kupplungsabschnitt (14) der Griffzubehörteilen (12.1, 12.2, ... , 12.n).

9. Vorrichtung (1) nach einem der vorhergehenden Ansprüche, wobei die verstellbare Kupplung (6) dazu konfiguriert ist, dass der Hauptkörper (5) in Bezug auf die Verbindungsbasis (2) um eine Hauptverstellungsachse (26) drehbar ist und in Bezug auf die Verbindungsbasis (2) verriegelbar ist, wobei die Hauptverstellungsachse (26) in Bezug auf die erste Drehachse (15') und in Bezug auf die zweite Drehachse (15") geneigt ist.

10. Vorrichtung (1) nach Anspruch 9, wobei die Hauptverstellungsachse (26) in Bezug auf die Drehachsen (15', 15") in einem Winkel zwischen 45° und 65°, oder zwischen 50° und 60°, oder 54,7°, geneigt ist, sodass, mittels einer Drehverstellung ausschließlich um die Hauptverstellungsachse (26), die Orientierung der ersten (15') und der zweiten (15") Drehachsen in dem Raum entlang drei Richtungen, die zueinander orthogonal sind, ermöglicht wird.

11. Vorrichtung (1) nach einem der vorhergehenden Ansprüche, wobei die Griffzubehörteile (12.1, ... , 12.n) zwei Lenkerzubehörteile (12.1) umfassen, die jeweils in der Lage sind, mit einer der ersten (8') und der zweiten (8") Drehschnittstellen zu koppeln, wobei der Griffabschnitt (13) in Bezug auf den Kupplungsabschnitt (14) um eine Lenkerorientierungsachse (27), die orthogonal zu der ersten Drehachse (15') und zu der zweiten Drehachse (15") ist, in Position orientiert und verriegelt werden kann, wenn das Lenkerzubehörteil (12.1) mit der Drehschnittstelle (8', 8") gekoppelt wird.

12. Vorrichtung (1) nach einem der vorhergehenden Ansprüche, wobei die Griffzubehörteile (12.1, ... , 12.n) ein Radzubehörteil (12.2) umfassen, das in der Lage ist, mit einer der ersten (8') und der zweiten (8") Drehschnittstellen gekoppelt zu werden und das zwei voneinander beabstandete Griffabschnitte (13) aufweist, die auf zwei gegenüberliegenden Seiten in Bezug auf die erste (8') oder die zweite (8") Drehschnittstelle positioniert sind und in Bezug auf den Kupplungsabschnitt (14) um jeweilige Lenkerorientierungsachsen (27, 27) in Position verstellbar und verriegelbar sind.

13. Vorrichtung (1) nach einem der vorhergehenden Ansprüche, wobei die Griffzubehörteile (12.1, ... , 12.n) zwei Kurbelzubehörteile (12.3) umfassen, die jeweils mit einer der ersten (8') und der zweiten (8") Drehschnittstellen gekoppelt sind, wobei sich der Griffabschnitt (13) parallel und beabstandet in Bezug auf die erste Drehachse (15') und in Bezug auf die zweite Drehachse (15") erstreckt.

14. Vorrichtung (1) nach einem der vorhergehenden Ansprüche, wobei die Griffzubehörteile (12.1, ... , 12.n) ein Verbindungszubehörteil (12.4) umfassen, das zwei Kupplungsabschnitte (14) aufweist, die beide über zwei gelenkige Verbindungen mit einem einzigen Griffabschnitt (13) verbunden sind.

15. Vorrichtung (1) nach einem der vorhergehenden Ansprüche, wobei die Griffzubehörteile (12.1, ... , 12.n) ein Verschiebungszubehörteil (12.5) umfassen, dessen Griffabschnitt (13) linear in Bezug auf den Kupplungsabschnitt (14) verschiebbar ist und das einen nicht selbsthemmenden Bewegungsumwandlungsmechanismus (30), beispielsweise eine Zahnstange umfasst, der zwischen dem Griffabschnitt (13) und dem Kupplungsabschnitt (14) angeordnet ist, sowie Drehverhinderungsmittel (31), die eine Drehung des Griffabschnitts (13) in Bezug auf den Hauptkörper (5) verhindern.

16. Vorrichtung (1) nach einem der vorhergehenden Ansprüche, wobei die Kupplungsabschnitte (14) der Griffzubehörteile (12.1, ... , 12.n) eine sichere Winkelreferenz für eine Verbindung mit der Drehschnittstelle (8', 8") in einer einzigen relativen Winkelposition bilden.

17. Vorrichtung (1) nach einem der vorhergehenden Ansprüche, wobei die Steuervorrichtung (11):

 - einen Computer umfasst, das mit einer Benutzerschnittstelle (32) mit einer Anzeige (33) verbunden werden kann;
 - dazu konfiguriert ist, die Auswahl einer Mehrzahl von Rehabilitationsprogrammen mit der Möglichkeit des Einstellen einer Mehrzahl von Bewegungsparameter des jeweiligen Rehabilitationsprogramms zu ermöglichen,

 wobei die Rehabilitationsprogramme Rehabilitationsprogramme mit Bewegungen der ersten Drehschnittstelle (8') und der zweiten Drehschnittstelle (8") umfassen, die nicht miteinander synchronisiert sind.

18. Vorrichtung (1) nach einem der vorhergehenden Ansprüche, wobei die Steuervorrichtung (11) dazu konfiguriert ist, um:

 - Rehabilitationsprogramme mit einer Steuerung des ersten Motors (7') und des zweiten Motors (7") mittels einer Winkelpositionssteuerung auszuführen,
 - Rehabilitationsprogramme mit einer Steuerung des ersten Motors (7') und des zweiten Motors (7") mittels einer Drehgeschwindigkeitssteuerung auszuführen.

19. Vorrichtung (1) nach einem der vorhergehenden Ansprüche, wobei die Steuervorrichtung (11) dazu konfiguriert ist, Rehabilitationsprogramme mit einer Steuerung des ersten Motors (7') und des zweiten Motors (7")mittels einer Impedanz- und/oder Admittanzsteuerung auszuführen, basierend auf:

 - Winkelpositionen der ersten Drehschnittstelle (8') und der zweiten Drehschnittstelle (8"), die durch die ersten Positionserkennungsmittel (9') und die zweiten Positionserkennungsmittel (9"),
 - theoretischen Winkelpositionen der ersten Drehschnittstelle (8') und der zweiten Drehschnittstelle (8"), die durch die Steuervorrichtung (11) gesteuert werden,
 - den entsprechenden Drehmomenten, die auf die erste Drehschnittstelle (8') und die zweite Drehschnittstelle (8") übertragen werden, die durch die ersten Drehmomenterkennungsmittel (10') und die zweiten Drehmomenterkennungsmittel (10") erkannt werden.

20. Vorrichtung (1) nach einem der vorhergehenden Ansprüche, wobei die Steuervorrichtung (11) dazu konfiguriert ist, eine Steuerung der Bewegung des ersten Motors (7') und des zweiten Motors (7") in Abhängigkeit von einem oder mehreren Abhängigkeitsparametern und Abhängigkeitsfunktionen auszuführen, die vordefiniert sind oder über eine Benutzerschnittstelle (32) ausgewählt werden können.

21. Vorrichtung (1) nach einem der vorhergehenden Ansprüche, wobei die Steuervorrichtung (11) in Signalverbindung mit einem oder mehreren der folgenden Elemente steht:

 - einem oder mehreren elektromiographischen Sensoren (35),
 - einem oder mehreren funktionellen elektrischen Stimulationsvorrichtungen (36),
 - einem oder mehreren EEG-Signaldetektoren (37),
 - einer oder mehreren elektronischen graphischen (33) und/oder akustischen (38) Benutzerschnittstellen,
 - einem oder mehreren EDA-Sensoren (39),
 - einem oder mehreren EKG-Sensoren (40),
 - einer oder mehreren Digitalkameras (41),

 und dazu konfiguriert ist, den ersten Motor (7') und den zweiten Motor (7") in Abhängigkeit von den Signalen, die dadurch bereitgestellt werden, zu steuern.

22. Vorrichtung (1) nach einem der vorhergehenden Ansprüche, wobei sowohl die erste (15') als auch die zweite (15") Drehachse eine feste relative Position zueinander aufweisen.

**Revendications**

1. Un dispositif (1) pour la rééducation bilatérale des membres d'un utilisateur, comprenant :

 - une base de connexion (2) pour une connexion fixe de la base de connexion (2) à un support (4),
 - un corps principal (5) relié à la base de connexion (2),
 - un premier moteur (7') et un second moteur (7") supportés par le corps principal (5),
 - une première interface rotative (8') supportée par le corps principal (5) en une première position et pouvant être actionnée en rotation autour d'un premier axe de rotation (15') par le premier moteur (7'), et
 - une seconde interface rotative (8") supportée par le corps principal (5) en une seconde position espacée de la première position et pouvant être actionnée en rotation autour d'un second axe de rotation (15") par le second moteur (7"),
- un ensemble (12) d'accessoires de préhension (12.1, 12.2, ... , 12.n) comprenant chacun une portion de préhension (13) et une portion de couplage (14) qui sont réversiblement couplables et solidaires en rotation à l'une première interface rotative (8') et seconde interface rotative (8"), respectivement,
- un dispositif de commande électronique (11) en connexion de signal avec le premier moteur (7') et avec le second moteur (7") et comprenant :
- des premiers moyens de détection de position (9') pour détecter une position angulaire de la première interface rotative (8') par rapport au corps principal (5),
- des seconds moyens de détection de position (9") pour détecter une position angulaire de la seconde interface rotative (8") par rapport au corps principal (5),
dans lequel le dispositif de commande électronique (11) est configuré pour commander les mouvements rotatifs appliqués aux première interface rotative (8') et une seconde interface rotative (8") en fonction des positions angulaires détectées par les premiers et seconds moyens de détection de position (9', 9"), **caractérisé en ce que**
le corps principal (5) est relié à la base de connexion (2) par une articulation (6) réglable et verrouillable permettant un réglage et un verrouillage de la position du corps principal (5) par rapport à la base de connexion (2) dans au moins trois positions différentes du corps avec différentes orientations du premier (15') et du second (15") axes de rotation.

2. Un dispositif (1) selon la revendication 1, dans lequel le dispositif de commande électronique (11) comprend en outre :

- des premiers moyens de détection de couple (10') pour détecter un couple appliqué à la première interface rotative (8'),
- des seconds moyens de détection de couple (10") pour détecter un couple appliqué à la seconde interface rotative (8"),

dans lequel le dispositif de commande électronique (11) est configuré pour commander les mouvements rotatifs et les couples appliqués à 1 première interface rotative (8') et à la seconde interface rotative (8") en fonction des positions angulaires détectées par le premier moyen et seconds moyen de détection de position (9', 9") et/ou des couples détectés par les premiers et seconds moyens de détection de couple (10', 10").

3. Un dispositif (1) selon l'une quelconque des revendications précédentes, dans lequel la base de connexion (2) comprend une forme en « U » ou de type pince définissant un siège de connexion (16) qui est latéralement accessible afin de pouvoir recevoir un bord d'une table.

4. Un dispositif (1) selon l'une quelconque des revendications précédentes, dans lequel la base de connexion (2) forme une portion de fixation articulée (17) pour fixer une portion de base (18) de l'articulation réglable (6) à la base de connexion (2), avec la possibilité d'un réglage rotatif de la position de base (18) par rapport à la base de connexion (2) autour d'un axe auxiliaire de réglage horizontal (19).

5. Un dispositif (1) selon l'une quelconque des revendications précédentes, dans lequel le corps principal (5) comprend un boîtier externe (22) qui accueille le premier moteur (7') et le second moteur (7"), dans lequel :

- le premier moteur (7') et le second moteur (7") sont disposés parallèlement l'un à l'autre et côte à côte, avec leurs extensions axiales se chevauchant, de sorte que la dimension axiale du premier moteur (7') et du second moteur (7") est inférieure à la somme de leurs extensions axiales individuelles,
- une première connexion opérationnelle du premier moteur (7') à la première interface rotative (8') et une seconde connexion opérationnelle du second moteur (7") à la seconde interface rotative (8") sont positionnées sur des côtés opposés du premier moteur (7') et du second moteur (7"), et
- la première connexion opérationnelle (23') est positionnée latéralement à côté du second moteur (7") avec leurs extensions axiales se chevauchant, et la seconde connexion opérationnelle (23") est positionnée latéralement à côté du premier moteur (7') avec leurs extensions axiales se chevauchant,
- la première interface rotative (8') et la seconde interface rotative (8") sont toutes deux en positions latéralement décalées par rapport au premier moteur (7') et au second moteur (7").

6. Un dispositif (1) selon l'une quelconque des revendications précédentes, dans lequel la première interface rotative (8') et la seconde interface rotative (8") sont accessibles depuis l'extérieur du corps principal (5) et font face à deux directions opposées vers l'extérieur du corps principal (5)a.

7. Un dispositif (1) selon l'une quelconque des revendications précédentes, dans lequel la première interface rotative (8') et la seconde interface rotative (8") sont positionnées sur deux côtés opposés du corps principal (5) et le premier axe de rotation (15')
et le second axe de rotation (15") sont parallèles ou
coïncidents.

8. Un dispositif (1) selon l'une quelconque des revendications précédentes, dans lequel la première interface rotative (8') et la seconde interface rotative
(8") comprennent des moyens de couplage rapide,
sans l'aide d'outils, ou l'un des éléments suivants :

   - un coupleur à encliquetage élastique,
   - un coupleur à écrou annulaire fileté,
   - un coupleur à baïonnette,
   - un coupleur à insertion,
   - un coupleur fileté,

   pour une connexion bloquée en rotation entre l'interface rotative (8', 8") et la portion de couplage (14)
   de l'accessoire de préhension (12.1, 12.2, ... , 12.n).

9. Un dispositif (1) selon l'une quelconque des revendications précédentes, dans lequel l'articulation réglable (6) est configurée de sorte que le corps principal (5) soit rotatif par rapport à la base de connexion (2) autour d'un axe principal de réglage (26) et
verrouillable par rapport à la base de connexion (2),
dans lequel l'axe principal de réglage (26) est incliné
par rapport au premier axe de rotation (15') et par
rapport au second axe de rotation (15").

10. Un dispositif (1) selon la revendication 9, dans lequel
l'axe principal de réglage (26) est incliné par rapport
aux axes de rotation (15', 15") d'un angle compris
entre 45° et 65°, ou entre 50° et 60°, ou 54,7°, de
manière à permettre, au moyen d'un réglage rotatif
autour du seul axe principal de réglage (26), l'orientation du premier axe de rotation (15') et du second
axes de rotation (15") dans l'espace selon trois directions orthogonales entre elles.

11. Un dispositif (1) selon l'une quelconque des revendications précédentes, dans lequel les accessoires
de préhension (12.1, ... , 12.n) comprennent deux
accessoires de type guidon (12.1) chacun apte à se
coupler à l'une première interface rotative (8') et
seconde interface rotative (8"), respectivement,
dans lequel la portion de préhension (13) est orientable et verrouillable en position par rapport à la
portion de couplage (14), autour d'un axe d'orientation du guidon (27) orthogonal au premier axe de
rotation (15') et au second axe de rotation (15")
lorsque l'accessoire guidon (12.1) est couplé à l'interface rotative (8', 8").

12. Un dispositif (1) selon l'une quelconque des revendications précédentes, dans lequel les accessoires
de préhension (12.1, ... , 12.n) comprennent un
accessoire de type roue (12.2) apte à se coupler à l'une première interface rotative (8') ou seconde
interface rotative (8") et comprenant deux portions
de préhension (13) espacées et positionnées sur
deux côtés opposés par rapport à la première (8')
ou seconde (8") interface rotative, et réglables et
verrouillables en position par rapport à la portion de
couplage (14), autour des axes d'orientation respectifs du guidon (27, 27).

13. Un dispositif (1) selon l'une quelconque des revendications précédentes, dans lequel les accessoires
de préhension (12.1, ... , 12.n) comprennent deux
accessoires de type manivelle (12.3) chacun couplé
respectivement à l'une première interface rotative
(8') et seconde interface rotative (8"), dans lequel la
portion de préhension (13) est parallèle et espacée
par rapport au premier axe de rotation (15') et par
rapport au second axe de rotation (15").

14. Un dispositif (1) selon l'une quelconque des revendications précédentes, dans lequel les accessoires
de préhension (12.1, ... , 12.n) comprennent un
accessoire de type liaison (12.4) comprenant lesdites deux portions de couplage (14) toutes deux
reliées à une seule portion de préhension (13) au
moyen de deux liaisons articulées.

15. Un dispositif (1) selon l'une quelconque des revendications précédentes, dans lequel les accessoires
de préhension (12.1, ... , 12.n) comprennent un
accessoire translationnel (12.5) dont la portion de
préhension (13) est translatable linéairement par
rapport à la portion de couplage (14), et qui
comprend un mécanisme de conversion de mouvement (30) non autobloquant, par exemple à crémaillère, interposé entre la portion de préhension (13) et
la portion de couplage (14), ainsi que des moyens
anti-rotation (31) qui empêchent une rotation de la
portion de préhension (13) par rapport au corps
principal (5).

16. Un dispositif (1) selon l'une quelconque des revendications précédentes, dans lequel les portions de
couplage (14) des accessoires de préhension
(12.1, ... , 12.n) forment une référence angulaire
sûre pour une connexion avec l'interface rotative
(8', 8") en une seule position angulaire relative.

17. Un dispositif (1) selon l'une quelconque des revendications précédentes, dans lequel le dispositif de
commande (11) :

   - comprend un ordinateur connectable à une
   interface utilisateur (32) avec un écran (33),
   - est configuré pour permettre la sélection d'une
   pluralité de programmes de rééducation avec la
   possibilité de régler une pluralité de paramètres
   de mouvement du programme de rééducation correspondant,

dans lequel les programmes de rééducation comprennent des programmes de rééducation avec des mouvements de la première interface rotative (8') et de la seconde interface rotative (8") qui ne sont pas synchronisés l'un avec l'autre.

18. Un dispositif (1) selon l'une quelconque des revendications précédentes, dans lequel le dispositif de commande (11) est configuré pour exécuter :

- des programmes de rééducation avec une commande du premier moteur (7') et du second moteur (7") au moyen d'une commande de position angulaire,
- des programmes de rééducation avec une commande du premier moteur (7') et du second moteur (7") au moyen d'une commande de vitesse de rotation.

19. Un dispositif (1) selon l'une quelconque des revendications précédentes, dans lequel le dispositif de commande (11) est configuré pour exécuter des programmes de rééducation avec une commande du premier moteur (7') et du second moteur (7") au moyen d'une commande d'impédance et/ou d'admittance, basée sur :

- les positions angulaires de la première interface rotative (8') et de la seconde interface rotative (8") détectées par les premiers moyens de détection de position (9') et les seconds moyens de détection de position (9"),
- les positions angulaires théoriques de la première interface rotative (8') et de la seconde interface rotative (8") commandées par le dispositif de commande (11), et
- les couples correspondants transmis à la première interface rotative (8') et à la seconde interface rotative (8"), détectés par les premiers moyens de détection de couple (10') et les seconds moyens de détection de couple (10").

20. Un dispositif (1) selon l'une quelconque des revendications précédentes, dans lequel le dispositif de commande (11) est configuré pour une commande du mouvement du premier moteur (7') et du second moteur (7") de manière dépendante, en fonction d'un ou plusieurs paramètres de dépendance et fonctions de dépendance, prédéfinis ou sélectionnables par une interface utilisateur (32).

21. Un dispositif (1) selon l'une quelconque des revendications précédentes, dans lequel le dispositif de commande (11) est en communication de signal avec un ou plusieurs des éléments suivants :

- un ou plusieurs capteurs électromyographiques (35),
- un ou plusieurs dispositifs de stimulation électrique fonctionnelle (36),
- un ou plusieurs détecteurs de signaux EEG (37),
- une interface rotative ou plusieurs interfaces utilisateur électroniques visuelles (33) et/ou auditives (38),
- un ou plusieurs capteurs EDA (39),
- un ou plusieurs capteurs ECG (40),
- une ou plusieurs caméras numériques (41),

et est configuré pour commander le premier moteur (7') et le second moteur (7") en fonction des signaux fournis par ceux-ci.

22. Un dispositif (1) selon l'une quelconque des revendications précédentes, dans lequel le premier axe de rotation (15') et second axe de rotation (15") ont une position relative fixe l'un par rapport à l'autre.

FIG. 1

FIG. 2

FIG. 3

FIG. 4

FIG. 5

FIG. 6

FIG. 7

EP 4 518 825 B1

FIG. 8A    FIG. 8B    FIG. 8C

FIG. 9

FIG. 10.1

FIG. 10.2

FIG. 10.3

FIG. 10.4

24

FIG. 10.5

FIG. 10.6

FIG. 10.7

FIG. 10.9

FIG. 10.8

FIG. 11

FIG. 12

FIG. 13

FIG. 14.1

FIG. 14.2

FIG. 14.3

FIG. 14.4

FIG. 14.5

FIG. 14.6

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1734912 B1